# EUROPEAN PATENT APPLICATION

(11) **EP 3 586 885 A1**
(43) Date of publication of application: **01.01.2020**
(21) Application number: 19182121.4
(22) Date of filing: 25.06.2019
(51) Int. Cl.: A61L 27/36

(54) **DECELLULARIZED TISSUE PRODUCING METHOD AND DECELLULARIZED TISSUE PRODUCING APPARATUS**

(30) Priority: 29.06.2018 JP 2018125168; 29.06.2018 JP 2018125169; 04.09.2018 JP 2018165409; 04.09.2018 JP 2018165407
(71) Applicant: Ricoh Company, Ltd., Tokyo 143-8555 (JP)
(72) Inventor: SHINOHARA, Satoshi, Tokyo, 143-8555 (JP); SUZUKI, Shogo, Tokyo, 143-8555 (JP)
(74) Representative: SSM Sandmair

(57) **Abstract**

A decellularized tissue producing method includes degrading DNA included in a biological tissue in which cells are destroyed, by using a first treatment liquid; degreasing the biological tissue in which the DNA is degraded, by using a second treatment liquid; and washing the degreased biological tissue, by using a third treatment liquid. At least one of the degrading, the degreasing, and the washing is performed by a circulation method.

## Description

### BACKGROUND OF THE INVENTION

### 1. Field of the Invention

The present invention relates to a decellularized tissue producing method and a decellularized tissue producing apparatus.

### 2. Description of the Related Art

In regenerative medicine, as a support tissue to regenerate a patient's organ that has become deficient, decellularized tissue has been re-transplanted. This decellularized tissue is obtained by removing, from biological tissue of a human or a heterologous mammal, cellular components such as cytoplasmic components, cytosol components, cytoskeletons, and cell membrane components. The decellularized tissue is mainly formed of extracellular matrix components such as elastin, collagen (type I, type IV, etc.), and laminin.

Patent Document 1 discloses a method of producing a decellularized tissue including the steps of: destroying cells in a biological tissue by using an aqueous solution of polyethylene glycol; degrading the DNA included in the biological tissue, in which the cells have been destroyed, by using DNase; and washing the biological tissue, in which the DNA has been degraded, by using phosphate buffered saline (PBS) (see, for example, Patent Document 1).

Patent Document 1: Japanese Translation of PCT International Application Publication No. JP-T-2005-531355

The present disclosure has an object to provide a decellularized tissue producing method and a decellularized tissue producing apparatus, by which a decellularized tissue can be efficiently produced.

### SUMMARY OF THE INVENTION

An aspect of the present invention provides a decellularized tissue producing method and a decellularized tissue producing apparatus, in which one or more of the disadvantages of the related art are reduced.

According to one aspect of the present invention, there is provided a decellularized tissue producing method including degrading DNA included in a biological tissue in which cells are destroyed, by using a first treatment liquid; degreasing the biological tissue in which the DNA is degraded, by using a second treatment liquid; and washing the degreased biological tissue, by using a third treatment liquid, wherein at least one of the degrading, the degreasing, and the washing is performed by a circulation method.

According to one embodiment of the present invention, a decellularized tissue producing method and a decellularized tissue producing apparatus by which a decellularized tissue can be efficiently produced, can be provided.

### BRIEF DESCRIPTION OF THE DRAWINGS

FIG. 1 is a flow chart illustrating an example of a decellularized tissue producing method according to a first embodiment of the present invention;
FIG. 2 is a schematic diagram illustrating an example of a decellularized tissue producing apparatus according to the first embodiment of the present invention;
FIG. 3 is a schematic diagram illustrating a cell destruction apparatus used in an example in the first embodiment of the present invention;
FIG. 4 is a schematic diagram illustrating a decellularized tissue producing apparatus used in an example in the first embodiment of the present invention;
FIG. 5 is a flow chart illustrating an example of a decellularized tissue producing method according to a second embodiment of the present invention; and
FIGS. 6A and 6B illustrate the result of the evaluation of the adhesion of cells to the decellularized tissue of practical example 1 and comparative example 1.

### DETAILED DESCRIPTION OF THE PREFERRED EMBODIMENTS

The related art described in the BACKGROUND OF THE INVENTION has a problem in that it is not possible to efficiently produce decellularized tissue, because it takes a long time to degrade the DNA included in the biological tissue in which the cells have been destroyed, and to wash the biological tissue, in which the DNA has been degraded, by using PBS.

The related art described in the BACKGROUND OF THE INVENTION has another problem in that the resulting decellularized tissue (support tissue) includes residual lipophilic components, and, therefore, even when cells are to be adhered to the decellularized tissue (support tissue), it is difficult for the cells to adhere to the decellularized tissue (support tissue).

Hereinafter, embodiments of the present invention will be described with reference to the drawings.

### <First embodiment>

### (Decellularized tissue producing method -first embodiment)

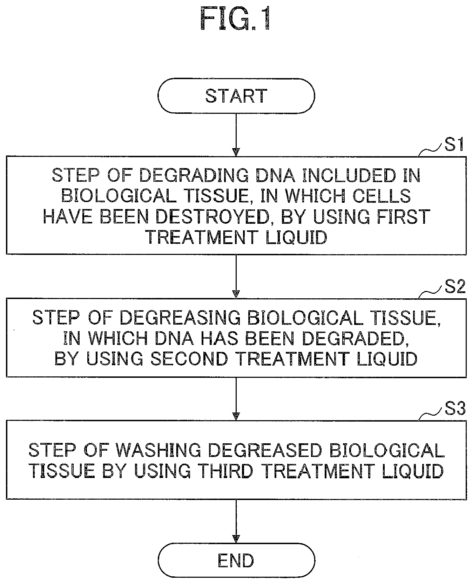

FIG. 1 illustrates an example of a decellularized tissue producing method according to the present embodiment.

A decellularized tissue producing method includes the steps of: degrading the DNA included in the biological tissue, in which the cells have been destroyed, by using a first treatment liquid (step S1); degreasing the biological tissue, in which the DNA has been degraded, by using a second treatment liquid (step S2); and washing the degreased biological tissue by using a third treatment liquid (step S3), and at least one of steps S1, S2, and S3 is performed by a circulation method. Accordingly, a decellularized tissue can be efficiently produced.

In the present specification and claims, the circulation method is a method in which a treatment liquid flows into a contact tank and the biological tissue is treated by the treatment liquid, when the treatment liquid flows out of the contact tank, that is, the circulation method is a method in which the biological tissue is treated by the treatment liquid, when the treatment liquid circulates through the contact tank.

In step S1, for example, a liquid including an enzyme or a surfactant is brought into contact with the biological tissue in which the cells have been destroyed, to degrade the DNA included in the biological tissue in which the cells have been destroyed.

The enzyme may be, but is not limited to, DNase (e.g., DNaseI), trypsin, and the like.

The surfactant may be, but is not limited to, an ionic surfactant (e.g., sodium dodecyl sulfate (SDS)), a non-ionic surfactant (e.g., Triton X-100), and the like.

The DNase may be, for example, DNaseI.

The liquid is not particularly limited as long as the liquid is physiologically compatible, may be a liquid in which water is the main solvent such as physiological saline, phosphate buffered saline (PBS), etc., and two or more kinds of liquids may be used in combination. Among these, physiological saline is preferable.

The method of bringing a liquid including DNase into contact with a biological tissue in which the cells have been destroyed may be, but is not limited to, a method of mixing and stirring a biological tissue in which the cells have been destroyed with a liquid including DNase, and a method of causing a liquid including DNase to circulate through a biological tissue in which the cells have been destroyed, etc. Among these, it is preferable to perform the method of causing a liquid including DNase to circulate through a biological tissue in which the cells have been destroyed, in that the DNA can be efficiently degraded.

When the biological tissue, in which the cells have been destroyed, is mixed and stirred with a liquid including DNase, the liquid including DNase may be appropriately replaced.

Preferably, the temperature of the environment in which the liquid including DNase is brought into contact with the biological tissue in which the cells have been destroyed, is preferably 4 °C to 40 °C. When the temperature of the environment in which the liquid including DNase is brought into contact with the biological tissue in which the cells have been destroyed, is 4 °C or higher, it is possible to inhibit damage due to ice crystals of an extracellular matrix included in the biological tissue, and when the temperature is 40 °C or lower, it is possible to inhibit denaturation of the protein included in the biological tissue.

Further, it is more preferable that the temperature of the environment in which the liquid including DNase is brought into contact with the biological tissue in which the cells have been destroyed, is 25 °C to 37 °C. In general, DNase exhibits high activity in the range of 25 °C to 37 °C, and can efficiently degrade the DNA.

In step S2, for example, the biological tissue in which the DNA has been degraded in step S1 is brought into contact with a liquid including an organic solvent, to degrease the biological tissue in which the DNA has been degraded.

In the present specification and claims, degreasing is defined as removing lipid-soluble components from the biological tissue.

The organic solvent is not particularly limited as long as the organic solvent is a physiologically acceptable organic solvent capable of degreasing the biological tissue in which the DNA has been degraded, and the organic solvent may be ethanol and the like, and two or more types of organic solvents may be used in combination. Among these, ethanol is preferable in that ethanol is harmless to the biological tissue.

The liquid is not particularly limited as long as the liquid is physiologically compatible, may be a liquid in which water is the main solvent such as physiological saline, phosphate buffered saline (PBS), etc., and two or more kinds of liquids may be used in combination. Among these, physiological saline is preferable.

The method of bringing the biological tissue in which the DNA has been degraded into contact with a liquid including an organic solvent may be, but is not limited to, mixing and stirring the biological tissue in which the DNA has been degraded with a liquid including an organic solvent, and a method of circulating a liquid including an organic solvent through the biological tissue in which the DNA has been degraded. Among these, the method of circulating a liquid including an organic solvent through the biological tissue in which the DNA has been degraded is preferable, in that the biological tissue can be efficiently degreased.

When the biological tissue in which the DNA has been degraded is mixed and stirred with a liquid including an organic solvent, the liquid including the organic solvent may be appropriately replaced.

The temperature of the environment in which the biological tissue in which the DNA has been degraded is brought into contact with a liquid including an organic solvent, is preferably 4 °C to 40 °C. When the temperature of the environment in which the biological tissue in which the DNA has been degraded is brought into contact with a liquid including an organic solvent, is 4 °C or higher, it is possible to inhibit damage due to ice crystals of an extracellular matrix included in the biological tissue, and when the temperature is 40 °C or lower, it is possible to inhibit denaturation of the protein included in the biological tissue.

Steps S1 and S2 may be performed simultaneously, or the order of steps S1 and S2 may be reversed.

In step S3, for example, the biological tissue degreased in step S2 is brought into contact with liquid, to wash the degreased biological tissue.

In the present specification and claims, washing is defined as removing water-soluble components from biological tissue.

The liquid is not particularly limited as long as the liquid is physiologically compatible, and may be a liquid in which water is the main solvent such as physiological saline, phosphate buffered saline (PBS), etc., and two or more kinds of liquids may be used in combination. Among these, physiological saline is preferable.

The method of bringing the degreased biological tissue into contact with a liquid may be, but is not limited to, mixing and stirring the degreased biological tissue with a liquid, and a method of causing the liquid to circulate through the degreased biological tissue. Among these, the method of causing the liquid to circulate through the degreased biological tissue is preferable in that the degreased biological tissue can be efficiently cleaned.

When the biological tissue in which the DNA has been degraded is mixed and stirred with a liquid, the liquid may be appropriately replaced.

The temperature of the environment in which the degreased biological tissue is brought into contact with the liquid, is preferably 4 °C to 40 °C. When the temperature of the environment in which the degreased biological tissue is brought into contact with the liquid, is 4 °C or higher, it is possible to inhibit damage due to ice crystals of an extracellular matrix included in the biological tissue, and when the temperature is 40 °C or lower, it is possible to inhibit denaturation of the protein included in the biological tissue.

The method of producing the decellularized tissue of the present embodiment may further include the step of dissolving the cell membrane component of the biological tissue and destroying the cells of the biological tissue, as a pre-step of the step of degrading the DNA included in the biological tissue in which the cells have been destroyed.

### (Method of destroying cells in biological tissue-first embodiment)

The method of destroying cells in the biological tissue may be, but is not limited to, a method of bringing the biological tissue and a liquid including liquefied gas into contact with each other, a mechanical method (an ultrasonic method, a microbeads method, a freezing method, etc.) (e.g., Japanese Translation of PCT International Application Publication No. JP-T-2016-523541), a chemical method (using acids, alkalis, surfactants, etc.), a method of combining a mechanical method and a chemical method, a method of permeating the biological tissue with a supercritical carbon dioxide (e.g., Japanese Unexamined Patent Application Publication No. H6-218036), and the like. Among these, the method of bringing the biological tissue and a liquid including liquefied gas into contact with each other is preferable, in that the decellularized tissue is substantially not damaged and residual liquefied gas is reduced.

Here, a liquid including liquefied gas dissolves the cell membrane components, and can therefore destroy cells of the biological tissue.

In the present specification and claims, liquefied gas is a substance that is a gas in a standard state (0 °C, 1 atm (0.101325 MPa) .

The liquefied gas is not limited as long as the cells in the decellularized tissue can be destroyed, and the liquefied gas may be dimethyl ether, ethylmethyl ether, formaldehyde, ketene, acetaldehyde, propane, butane, liquefied petroleum gas, and the like, and two or more of these types may be used in combination. Among these, ethylmethyl ether and dimethyl ether are preferable in being liquefied at relatively low temperature and low pressure, and dimethyl ether is particularly preferable.

Dimethyl ether is liquefied at approximately 1 °C to 40 °C and 0.2 MPa to 5 MPa, and, therefore, the cost of the device will be low. Liquefied dimethyl ether vaporizes readily in the standard state, and is therefore unlikely to remain in the decellularized tissue.

When bringing the biological tissue and a liquid including liquefied gas into contact with each other, this is performed in an environment in which the pressure is greater than or equal to the saturated vapor pressure, such as in a gas-tight extraction tank, in order to maintain the liquid state of the liquefied gas.

The method of bringing the biological tissue and a liquid including liquefied gas into contact with each other may be, but is not limited to, a method of immersing the biological tissue in a liquid including liquefied gas, and a method of circulating a liquid including liquefied gas through the biological tissue. Among these, the method of circulating a liquid including liquefied gas through the biological tissue is preferable in that the cells can be efficiently destroyed.

The liquid including liquefied gas may further include a solvent.

The solvent may be, but is not limited to, ethanol, water, physiological saline, phosphate buffered saline (PBS), and the like, and two or more types of solvents may be used in combination.

The additive amount of solvent is preferably less than or equal to the solubility in the liquefied gas. Accordingly, the liquid including liquefied gas can be made uniform.

The temperature of the liquefied gas is preferably 1 °C to 40 °C, and more preferably 10 °C to 30 °C.

The pressure of the liquefied gas is preferably 0.2 MPa to 5 MPa, and more preferably in the range of 0.3 MPa to 0.7 MPa.

After the biological tissue is brought into contact with a liquid including liquefied gas, the temperature and pressure are returned to the standard state, and then the liquefied gas evaporates, so the liquefied gas can be easily removed.

The step of bringing the biological tissue into contact with a liquid including liquefied gas, may be repeated a plurality of times.

### (Biological tissue-first embodiment)

A biological tissue is defined as a tissue obtained from any one of a plant, fungi, archaea, and eubacteria having cell walls, or an animal without cell walls. Examples of the biological tissue are, but are not limited to, leaves, branches, trees, petals, stems, roots, pulp, pericarp, and seeds; soft tissue including skin, blood vessels, heart valves, cornea, amnion, dura, and the like or portions thereof, organs including heart, kidney, liver, pancreas, brain, and the like or portions thereof, and bones, cartilage, tendon, and the like or portions thereof, derived from humans or heterogeneous mammals.

### (Decellularized tissue producing apparatus-first embodiment)

The decellularized tissue producing apparatus of the present embodiment includes: a first unit for degrading DNA included in the biological tissue, in which the cells have been destroyed, by using a first treatment liquid; a second unit for degreasing the biological tissue, in which the DNA is degraded, by using a second treatment liquid; and a third unit for washing the degreased biological tissue by using a third treatment liquid. As long as the circulation method is implemented by at least one of the first unit, the second unit, and the third unit, the unit implementing the circulation method is not particularly limited.

The decellularized tissue producing apparatus of the present embodiment preferably further includes: a first storage unit for storing the first treatment liquid; a second storage unit for storing the second treatment liquid; and a third storage unit for storing the third treatment liquid. Furthermore, it is preferable that the decellularized tissue producing apparatus of the present embodiment further includes: a first liquid feeding unit for feeding the first treatment liquid from the first storage unit to the contact tank; a second liquid feeding unit for feeding the second treatment liquid from the second storage unit to the contact tank; and a third liquid feeding unit for feeding the third treatment liquid from the third storage unit to the contact tank.

The decellularized tissue producing apparatus of the present embodiment preferably further includes: a cell destroying unit for destroying cells of the biological tissue by using a liquid including liquefied gas; a fourth storage unit for storing liquid including liquefied gas; and a fourth liquid feeding unit for feeding liquid including liquefied gas from the fourth storage unit to the contact tank. In this case, the cell destroying unit preferably implements the circulation method.

Furthermore, the decellularized tissue producing apparatus of the present embodiment preferably further includes: a contact unit for bringing each treatment liquid that has been fed and a biological tissue in which the cells have been destroyed in contact with each other; a collecting unit for collecting the treatment liquid in contact with the biological tissue in which the cells have been destroyed; a separating unit for removing impurities mixed into the collected treatment liquid; and a fifth liquid feeding unit for feeding the treatment liquid to the separating unit from the collecting unit.

Furthermore, the decellularized tissue producing apparatus of the present embodiment preferably further includes a temperature detecting and adjusting unit for detecting and adjusting the internal temperature.

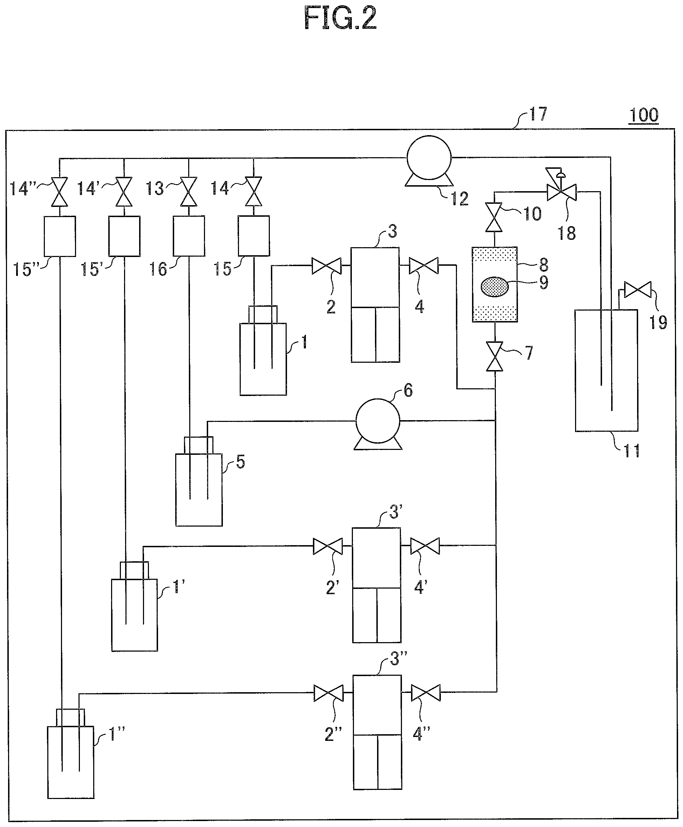

FIG. 2 illustrates an example of a decellularized tissue producing apparatus of the present embodiment.

A decellularized tissue producing apparatus 100 includes a tank 1 for storing a first treatment liquid, a tank 5 for storing a second treatment liquid, a tank 1' for storing a third treatment liquid, and a tank 1" for storing a liquid including liquefied gas. The decellularized tissue producing apparatus 100 also includes a pressurizing pump 3 (e.g., a syringe pump) for feeding the first treatment liquid, a pump 6 (e.g., a plunger pump) for feeding the second treatment liquid, a pressurizing pump 3' (e.g., a syringe pump) for feeding the third treatment liquid, and a pressurizing pump 3" (e.g., a syringe pump) for feeding the liquid including liquefied gas (e.g., a syringe pump).

The decellularized tissue producing apparatus 100 also includes a contact tank 8 for bringing each liquid treatment liquid that has been fed, into contact with a biological tissue 9 in which the cells have been destroyed, and a collection tank 11 for collecting each treatment liquid that has contacted the biological tissue 9 in which the cells have been destroyed.

With respect to the circulation velocity of the treatment liquid circulating through the contact tank 8, the velocity near the wall surface of the contact tank 8 and the velocity at the center part of the contact tank 8 may not be uniform, and the extraction efficiency may be inhibited. Accordingly, an end surface on an upstream side of the contact tank 8 may include a member that promotes equalizing the velocity distribution that occurs from near the wall surface of the contact tank 8 to the center of the contact tank 8.

The member that promotes equalizing the velocity distribution that occurs from near the wall surface of the contact tank 8 to the center portion of the contact tank 8, is not particularly limited, but a porous member having micro pores is preferable from the viewpoint of the efficiency of achieving uniformity of the velocity distribution.

Further, the decellularized tissue producing apparatus 100 includes separating units 15, 15', and 16 for removing impurities mixed into each treatment liquid that has been collected.

Furthermore, the decellularized tissue producing apparatus 100 includes a pump 12 (e.g., a plunger pump) for feeding the first treatment liquid from which impurities have been removed to the tank 1, feeding the second treatment liquid from which impurities have been removed to the tank 5, feeding the third treatment liquid from which impurities have been removed to the tank 1', or transporting the vaporized liquefied gas to a condenser 15".

Further, the decellularized tissue producing apparatus 100 includes a temperature detecting and adjusting unit 17 for detecting and adjusting the internal temperature.

Furthermore, the decellularized tissue producing apparatus 100 also includes valves 2, 2', 2", 4, 4', 4", 7, 10, 13, 14, 14', 14", and a backpressure valve 18.

Next, a method of producing a decellularized tissue using the decellularized tissue producing apparatus 100 will be described.

First, in step S1, the biological tissue 9, in which the cells have been destroyed, is introduced into the contact tank 8, and the first treatment liquid is filled into the tank 1. Next, the valves 2, 4, 7, and 10 are opened and the other valves are closed. Then, the first treatment liquid filled in the tank 1 is circulated through the contact tank 8 using the pressurizing pump 3. The DNA in the biological tissue 9 in which the cells have been destroyed, is degraded, for example, by the function of DNase. Next, the first treatment liquid including the DNA degradation product is collected in the collection tank 11. At this time, by using a pressurizing pump, it is possible to prevent the occurrence of cavitation in the first treatment liquid, in which the main solvent is water such that bubbles easily enter.

Next, the valve 14 is opened and the other valves are closed. Then, the collected first treatment liquid including the DNA degradation product, is fed to the tank 1 using the pump 12. At this time, the DNA degradation product is removed by the separating unit 15, so that the first treatment liquid from which the impurities have been removed, can be collected in the tank 1. The first treatment liquid collected in the tank 1 can be circulated directly to the contact tank 8 again to be used for DNA degradation of the biological tissue 9 in which the cells have been destroyed. Accordingly, the DNA can be degraded with a small amount of the first treatment liquid, without having to replace or add the first treatment liquid.

In step S2, the second treatment liquid is filled in the tank 5. Next, after the valves 7 and 10 are opened and the other valves are closed, the second treatment liquid filled in the tank 5 is circulated through the contact tank 8 using the pump 6, the biological tissue 9 in which the DNA has been degraded is degreased, and the second treatment liquid including the removed lipid is collected in the collection tank 11. Next, after the valve 13 is opened and the other valves are closed, the collected second treatment liquid including the lipid is fed to the tank 5 using the pump 12. At this time, the lipid is removed by the separating unit 16, and, therefore, the second treatment liquid from which impurities have been removed can be collected in the tank 5. The second treatment liquid collected in the tank 5 can be circulated directly back to the contact tank 8 again, to be used for degreasing the biological tissue 9 in which the DNA has been degraded. Therefore, it is possible to perform the degreasing with a small amount of the second treatment liquid, without having to replace or add the second treatment liquid.

In step S3, the third treatment liquid is filled in the tank 1'. Next, after the valves 2', 4', 7, and 10 are opened and other valves are closed, the third treatment liquid filled in the tank 1' is circulated through the contact tank 8 using the pressurizing pump 3', the degreased biological tissue 9 is washed, and the third treatment liquid including the removed water-soluble compound is collected in the collection tank 11. In this case, a pressurizing pump can be used to prevent the occurrence of cavitation in the third treatment liquid, in which the main solvent is water such that bubbles easily enter. Next, after the valve 14' is opened and the other valves are closed, the collected third treatment liquid including the water-soluble compound is fed to the tank 1' using the pump 12. In this case, the water-soluble compound is removed by the separating unit 15', and, therefore, the third treatment liquid from which the impurities have been removed can be collected in the tank 1'. The third treatment liquid collected in the tank 1' can be circulated directly back to the contact tank 8 again to be used for washing the degreased biological tissue 9. Therefore, washing can be performed with a small amount of the third treatment liquid, without having to replace or add the third treatment liquid.

Furthermore, the decellularized tissue producing apparatus 100 can use a liquid including liquefied gas to destroy cells of biological tissue, as will be described below. When cells are destroyed using a liquid including liquefied gas, the liquid including liquefied gas may become vaporized, which may damage the biological tissue due to drying. When liquid including liquefied gas is fed, the pressurizing pump 3" is used, and the pressure can be adjusted by the backpressure valve 18, and, therefore, it is possible to adjust or inhibit the vaporization of liquid including liquefied gas, thereby reducing damage to the biological tissue due to drying.

Note that cell destruction, DNA degradation, degreasing, and washing can be performed continuously by a circulation method by the following procedure.

First, the biological tissue 9 is introduced into the contact tank 8. Next, a liquid including liquefied gas is filled in the tank 1, the first treatment liquid is filled in the tank 1, the second treatment liquid is filled in the tank 5, and the third treatment liquid is filled in the tank 1'. Next, the valves 2", 4", 7, and 10 are opened, the backpressure valve 18 is adjusted to a predetermined pressure, the other valves are closed, and then, the liquid including liquefied gas filled in the tank 1" is fed by the pressurizing pump 3". Next, the pressurizing pump 3" is stopped, and the valves 2" and 4" are closed, and the liquid feeding is terminated. Next, a valve 19 is opened to reduce the pressure of the liquefied gas to less than a saturated vapor pressure, thereby vaporizing the liquefied gas present between the valve 4" and the valve 14". At this time, according to need, the vaporized dimethyl ether may be discharged by using a pump. Alternatively, instead of opening the valve 19, the valve 14" may be opened and the vaporized liquefied gas may be transported to the condenser 15" using the pump 12, to be liquefied and reused. Next, the valves 2, 4, 7, and 10 are opened and the other valves are closed. Then, the first treatment liquid filled in the tank 1 is fed by the pressurizing pump 3. At this time, by gradually reducing the set pressure of the backpressure valve 18 from the predetermined pressure described above to the atmospheric pressure, it is possible to inhibit the vaporization of the liquefied gas remaining in the contact tank 8, and it is possible to reduce the damage to the biological tissue 9 due to drying. Next, the pressurizing pump 3 is stopped, the valves 2 and 4 are closed, and liquid feeding is terminated. Next, the second treatment liquid filled in the tank 5 is fed by the pump 6. The pump 6 is then stopped and the liquid feeding is terminated. Next, the valves 2' and 4' are opened, and the third treatment liquid filled in the tank 1' is fed by the pressurizing pump 3'. Next, the pressurizing pump 3' is stopped, the valves 2', 4', 7, and 10 are closed, and the liquid feeding is terminated.

As described above, cell destruction, DNA degradation, degreasing, and washing can be performed continuously by a circulation method to shorten the treatment time.

### [[Practical examples-first embodiment]]

While practical examples of the present invention are described below, the present invention is not limited to the practical examples.

### [Practical example 1-first embodiment]

### (Cell destruction of swine-derived aortic tissue)

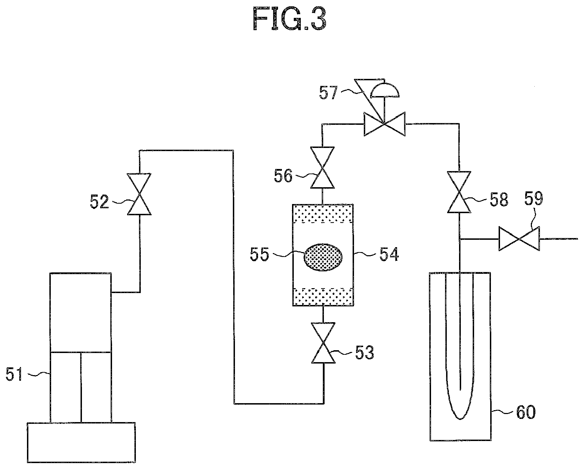

Cells of aortic tissue 55 derived from a swine were destroyed using a cell destruction apparatus illustrated in FIG. 3.

Specifically, the aortic tissue 55 derived from a swine was charged into an extraction tank 54. Next, the contents in a separation tank 60 were substituted in advance with dimethyl ether, and valves 52, 53, 56, 58, and 59 were closed. Next, the set pressure of a backpressure valve 57 was set to 0.7 MPa, the valves 52, 53, 56, and 58 were opened, the liquefied dimethyl ether was circulated by using a pump 51, and when the extraction tank 54 was filled with the liquefied dimethyl ether, the valves 53 and 56 were closed, the aortic tissue 55 derived from a swine was immersed in the liquefied dimethyl ether, and cell membrane components such as phospholipids were extracted. Next, the valves 53 and 56 were opened, and the flow rate of the liquefied dimethyl ether was adjusted to 1 mL/min, and the extraction liquid was collected in the separation tank 60. Next, the valve 58 was closed, the separation tank 60 was removed from the apparatus, and the liquefied dimethyl ether was volatilized in the draft chamber at atmospheric pressure.

By the above procedure, 60 mL of liquefied dimethyl ether and the aortic tissue 55 derived from a swine were brought into contact with each other, and then the valve 53 was closed, the valves 56, 58, and 59 were opened, the pressure in the extraction tank 54 was set to atmospheric pressure, and the liquefied dimethyl ether in the extraction tank 54 was volatilized and exhausted. As a result, the aortic tissue derived from a swine in which cells have been destroyed, was obtained.

### (DNA degradation, degreasing, and washing of swine-derived aortic tissue in which cells have been destroyed)

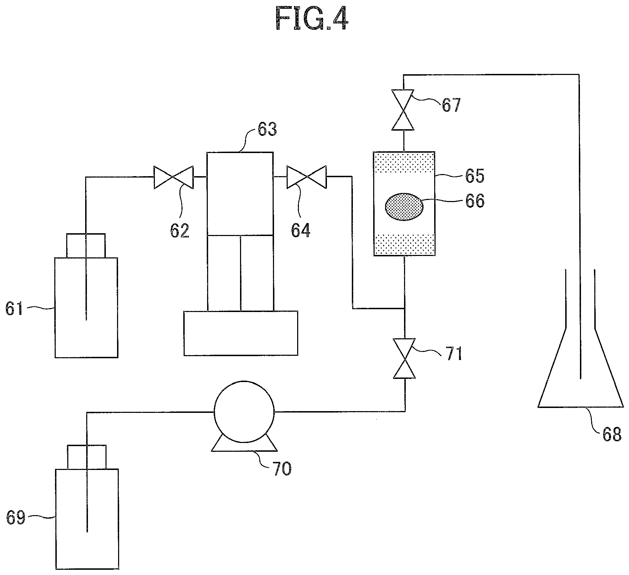

Aortic tissue 66 derived from a swine in which cells have been destroyed, was subjected to DNA degrading, degreasing, and washing by a circulation method using a decellularized tissue producing apparatus illustrated in FIG. 4.

Specifically, a contact tank 65 was charged with the aortic tissue 66 derived from a swine in which cells have been destroyed. Next, a tank 61 was filled with physiological saline including 0.2 mg/mL of DNaseI (manufactured by Roche Diagnostics K.K.) and 0.05 M of MgCl₂ (manufactured by Wako Pure Chemical Industries, Ltd.), and valves 62, 64, and 67 were opened and a valve 71 was closed. The physiological saline including DNaseI and MgCl₂ filled in the tank 61 was then circulated to the contact tank 65 at 1 mL/min using a syringe pump 63 to degrade the DNA included in the aortic tissue 66 derived from a swine in which cells have been destroyed, and the DNA degradation product was collected in a collection tank 68.

Next, in a tank 69, physiological saline including 80 volume% of ethanol was filled, and the valves 62 and 64 were closed and the valves 67 and 71 were opened. Next, the physiological saline including ethanol filled into the tank 69 was circulated to the contact tank 65 at 1 mL/min using a plunger pump 70, to degrease the aortic tissue 66 derived from a swine in which the DNA has been degraded, and the removed lipid was collected in the collection tank 68.

Next, the physiological saline including ethanol was discharged from the tank 69, and the tank 69 was filled with physiological saline. The physiological saline filled into the tank 69 was then circulated to the contact tank 65 at 1 mL/min using the plunger pump 70, and the degreased aortic tissue 66 derived from a swine was washed, and the removed extract was collected in the collection tank 68. As a result, a decellularized tissue of an aortic tissue derived from a swine was obtained.

### [Comparative example 1-first embodiment]

The DNA degradation, degreasing, and washing of the aortic tissue 66 derived from a swine in which cells have been destroyed, were performed by a batch method, i.e., except that the aortic tissue 66 was mixed and shaken with each treatment liquid, a decellularized tissue of an aortic tissue derived from a swine was obtained in the same manner as in practical example 1.

Table 1 indicates the amount of DNA per dry mass of decellularized tissue of an aortic tissue derived from a swine and an untreated aortic tissue derived from a swine.

**[Table 1]**

| | DNA DEGRADATION | DEGREASING | WASHING | DNA AMOUNT PER DRY MASS [ng/mg] |
|---|---|---|---|---|
| PRACTICAL EXAMPLE 1 | CIRCULATION METHOD | CIRCULATION METHOD | CIRCULATION METHOD | 360 |
| COMPARATIVE EXAMPLE 1 | BATCH METHOD | BATCH METHOD | BATCH METHOD | 730 |
| UNTREATED | - | - | - | 3370 |

Here, the DNA was extracted from the decellularized tissue of the aortic tissue derived from a swine and the untreated aortic tissue derived from a swine, by phenol-chloroform extraction. As for the DNA amount, the double-stranded DNA content was quantified using a Quant-iT PicoGreen dsDNA Assay Kit.

Table 1 indicates that the decellularized tissue of the aortic tissue derived from a swine of practical example 1 had a small amount of DNA per dry mass, and it was possible to efficiently produce the decellularized tissue.

In contrast, the decellularized tissue of the aortic tissue derived from a swine of comparative example 1 had a large amount of DNA per dry mass and was thus not efficiently produced, as a result of not subjecting the aortic tissue 66 derived from a swine in which cells have been destroyed to DNA degradation, degreasing, or washing by a circulation method.

### [Practical example 2-first embodiment]

Except that the degreasing and the washing were performed by a batch method, a decellularized tissue of an aortic tissue derived from a swine was obtained in the same manner as in practical example 1.

Specifically, the aortic tissue 66 derived from a swine in which the DNA has been degraded, was placed in physiological saline including 80 volume% of ethanol, and was then shaken and degreased. The degreased aortic tissue 66 derived from a swine was placed in physiological saline, and was then shaken and washed.

### [Practical example 3-first embodiment]

Except that the DNA degradation and the washing were performed by a batch method, a decellularized tissue of an aortic tissue derived from a swine was obtained in the same manner as in practical example 1.

Specifically, the aortic tissue 66 derived from a swine in which cells have been destroyed was placed in physiological saline including 0.2 mg/mL of DNaseI (manufactured by Roche Diagnostics K.K.) and 0.05 M of MgCl₂ (manufactured by Wako Pure Chemical Industries, Ltd.), and was shaken to degrade the DNA included in the aortic tissue 66 derived from a swine in which cells have been destroyed. The aortic tissue 66 derived from a swine in which the DNA has been destroyed, was placed in physiological saline, shaken, and washed.

### [Practical example 4-first embodiment]

Except that the DNA degradation and the degreasing were performed by a batch method, a decellularized tissue of an aortic tissue derived from a swine was obtained in the same manner as in practical example 1.

Specifically, the aortic tissue 66 derived from a swine in which cells have been destroyed was placed in physiological saline including 0.2 mg/mL of DNaseI (manufactured by Roche Diagnostics K.K.) and 0.05 M of MgCl₂ (manufactured by Wako Pure Chemical Industries, Ltd.), and was shaken to degrade the DNA included in the aortic tissue 66 derived from a swine in which cells have been destroyed. The aortic tissue 66 derived from a swine in which the DNA has been destroyed, was placed in physiological saline including 80 volume% of ethanol, and was shaken and degreased.

Table 2 indicates the residual amount of DNA, the residual amount of lipid, and the residual amount of water-soluble compounds in the decellularized tissue of the aortic tissue derived from a swine.

**[Table 2]**

| STEP | DNA DEGRADATION | | DEGREASING | | WASHING | |
|---|---|---|---|---|---|---|
| METHOD/INDEX | METHOD | INDEX | METHOD | INDEX | METHOD | INDEX |
| | | DNA RESIDUAL AMOUNT | | LIPID RESIDUAL AMOUNT | | WATER-SOLUBLE COMPOUND RESIDUAL AMOUNT |
| PRACTICAL EXAMPLE 1 | CIRCULATION METHOD | SMALL | CIRCULATION METHOD | SMALL | CIRCULATION METHOD | SMALL |
| PRACTICAL EXAMPLE 2 | CIRCULATION METHOD | SMALL | SHAKING METHOD | LARGE | SHAKING METHOD | LARGE |
| PRACTICAL EXAMPLE 3 | SHAKING METHOD | LARGE | CIRCULATION METHOD | SMALL | SHAKING METHOD | LARGE |
| PRACTICAL EXAMPLE 4 | SHAKING METHOD | LARGE | SHAKING METHOD | LARGE | CIRCULATION METHOD | SMALL |
| COMPARATIVE EXAMPLE 1 | SHAKING METHOD | LARGE | SHAKING METHOD | LARGE | SHAKING METHOD | LARGE |

In the decellularized tissue of the aortic tissue derived from a swine of practical example 1, similar to the residual amount of DNA, the residual amount of lipid and the residual amount of water-soluble compounds were also lower than those of the decellularized tissue of the aortic tissue derived from a swine of comparative example 1.

In the decellularized tissue of the aortic tissue derived from a swine of practical example 2, the DNA degrading was performed by a circulation method and the degreasing and the washing were performed by a batch method, and, therefore, the residual amount of DNA was lower than that of the decellularized tissue of the aortic tissue derived from a swine of comparative example 1.

In the decellularized tissue of the aortic tissue derived from a swine of practical example 3, the degreasing was performed by a circulation method and the DNA degrading and the washing were performed by a batch method, and, therefore, the residual amount of lipid was lower than that of the decellularized tissue of the aortic tissue derived from a swine of comparative example 1.

In the decellularized tissue of the aortic tissue derived from a swine of practical example 4, the washing was performed by a circulation method and the DNA degrading and the degreasing were performed by a batch method, and, therefore, the residual amount of water-soluble compounds was lower than that of the decellularized tissue of the aortic tissue derived from a swine of comparative example 1.

Thus, it can be seen that when at least one of the steps of DNA degrading, degreasing, and washing is performed by a circulation method, the decellularized tissue can be efficiently produced.

### <Second embodiment>

### (Decellularized tissue producing method -second embodiment)

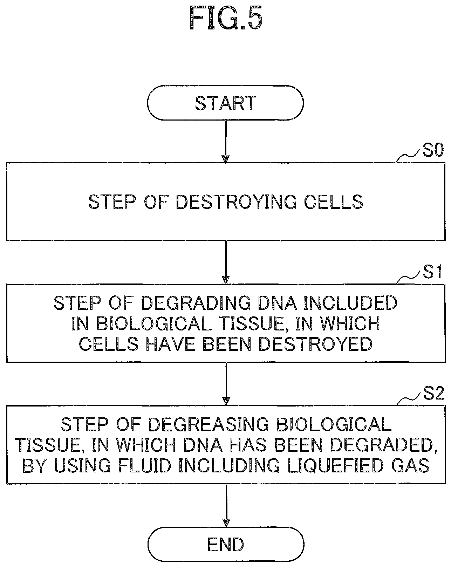

FIG. 5 illustrates an example of a decellularized tissue producing method of the second embodiment.

A decellularized tissue producing method includes the steps of: destroying cells in a biological tissue (step S0); degrading the DNA in the biological tissue in which the cells have been destroyed (step S1); and degreasing the biological tissue in which the DNA has been degraded using a fluid including liquefied gas (step S2). Accordingly, lipid-soluble components can be sufficiently removed from the biological tissue in which the DNA has been degraded, to improve the adhesion of cells to the decellularized tissue.

In the present specification and claims, degreasing is defined as removing lipid-soluble components from the biological tissue.

In step S0, for example, a liquid capable of destroying cells is brought into contact with biological tissue.

The liquid capable of destroying cells is preferably, but is not limited to, a liquid including a surfactant, high pressure water, liquefied dimethyl ether, or supercritical carbon dioxide.

In step S1, for example, a liquid capable of degrading DNA is brought into contact with the biological tissue in which the cells have been destroyed.

The liquid capable of degrading DNA is preferably, but is not limited to, a liquid including an enzyme or a surfactant.

The enzyme may be, but is not limited to, DNase (e.g., DNaseI), trypsin, and the like.

The surfactant may be, but is not limited to, an ionic surfactant (e.g., sodium dodecyl sulfate (SDS)), a non-ionic surfactant (e.g., Triton X-100), and the like.

The liquid is not particularly limited as long as the liquid is physiologically compatible, and the liquid may be physiological saline, phosphate buffered saline (PBS), and the like, and two or more kinds of liquids may be used in combination. Among these, physiological saline is preferable.

The method of bringing a liquid capable of degrading DNA into contact with a biological tissue in which the cells have been destroyed may be, but is not limited to, a method of mixing and stirring a biological tissue in which the cells have been destroyed with a liquid capable of degrading DNA, and a method of causing a liquid capable of degrading DNA to circulate through a biological tissue in which the cells have been destroyed, etc. Among these, it is preferable to perform the method of causing a liquid capable of degrading DNA to circulate through a biological tissue in which the cells have been destroyed, in terms of high efficiency of contact between the liquid capable of degrading DNA and the biological tissue in which the cells have been destroyed, and the DNA can be efficiently degraded.

When the biological tissue in which the cells have been destroyed is mixed and stirred with a liquid capable of degrading DNA, the liquid capable of degrading DNA may be appropriately replaced.

The temperature of the environment in which the liquid capable of degrading DNA is brought into contact with the biological tissue in which the cells have been destroyed, is preferably 4 °C to 40 °C. When the temperature of the environment in which the liquid capable of degrading DNA is brought into contact with the biological tissue in which the cells have been destroyed, is 4 °C or higher, it is possible to inhibit damage due to ice crystals of an extracellular matrix included in the biological tissue, and when the temperature is 40 °C or lower, it is possible to inhibit denaturation of the protein included in the biological tissue.

In step S2, for example, the fluid including liquefied gas is brought into contact with the biological tissue in which the DNA has been degraded in step S1.

In the present specification and claims, liquefied gas is a substance that is a gas in the standard state (0 °C, 1 atm (0.101325 MPa) .

The liquefied gas is not limited as long as the decellularized tissue can be degreased, and the liquefied gas may be dimethyl ether, ethylmethyl ether, formaldehyde, ketene, acetaldehyde, propane, butane, liquefied petroleum gas, and the like, and two or more of these types may be used in combination. Among these, ethylmethyl ether and dimethyl ether are preferable in being liquefied at relatively low temperature and low pressure, and dimethyl ether is particularly preferable.

Dimethyl ether is liquefied at approximately 1 °C to 40 °C and 0.2 MPa to 5 MPa, and, therefore, the cost of the device will be low. Liquefied dimethyl ether vaporizes readily in the standard state, and is therefore unlikely to remain in the decellularized tissue.

Step S2 is performed in an environment in which the pressure is greater than or equal to the saturated vapor pressure, such as in a gas-tight extraction tank, in order to maintain the liquid state of the liquefied gas.

The method of bringing a fluid including liquefied gas into contact with the biological tissue in which the DNA has been degraded may be, but is not limited to, a method of immersing the biological tissue in which the DNA has been degraded in a fluid including a liquefied gas.

The fluid including liquefied gas may further include a liquid different from the liquefied gas.

The liquid different from liquefied gas is not particularly limited as long as the liquid is physiologically compatible, and the liquid may be physiological saline, phosphate buffered saline (PBS), and the like, and two or more kinds of liquids may be used in combination. Among these, physiological saline is preferable.

The additive amount of the liquid different from liquefied gas is preferably less than or equal to the solubility in the liquefied gas. Accordingly, the fluid including the liquefied gas can be made uniform.

After the fluid including the liquefied gas is brought into contact with the biological tissue in which the DNA has been destroyed, the temperature and pressure are returned to the standard state, and then the liquefied gas evaporates, so the liquefied gas can be easily removed from the biological tissue in which the DNA has been destroyed.

The method of producing the decellularized tissue may further include a step of washing the degreased biological tissue (step S3).

In the present specification and claims, washing is defined as removing water-soluble components from biological tissue.

In step S3, for example, a liquid capable of washing the biological tissue is brought into contact with the biological tissue degreased in step S2.

The liquid capable of washing the biological tissue is not particularly limited as long as the liquid is physiologically compatible, and the liquid may be physiological saline, phosphate buffered saline (PBS), etc., and two or more kinds of liquids may be used in combination. Among these, physiological saline is preferable.

The method of bringing a liquid capable of washing the biological tissue into contact with the degreased biological tissue may be, but is not limited to, a method of mixing and stirring the degreased biological tissue with a liquid capable of washing the biological tissue, and a method of causing a liquid capable of washing the biological tissue to circulate through the degreased biological tissue, etc. Among these, it is preferable to perform the method of causing a liquid capable of washing the biological tissue to circulate through the degreased biological tissue, because the biological tissue can be efficiently washed.

When the degreased biological tissue is mixed and stirred with a liquid capable of washing the biological tissue, the liquid capable of washing the biological tissue may be appropriately replaced.

The temperature of the environment in which the liquid capable of washing the biological tissue is brought into contact with the degreased biological tissue, is preferably 4 °C to 40 °C. When the temperature of the environment in which the liquid capable of washing the biological tissue is brought into contact with the degreased biological tissue, is 4 °C or higher, it is possible to inhibit damage due to ice crystals of an extracellular matrix included in the biological tissue, and when the temperature is 40 °C or lower, it is possible to inhibit denaturation of the protein included in the biological tissue.

### (Method of destroying cells in biological tissue-second embodiment)

When destroying cells in the biological tissue, for example, a liquid capable of destroying the cells is brought into contact with the biological tissue.

The liquid capable of destroying cells is preferably, but is not limited to, a liquid including liquefied gas, a liquid including a surfactant, and the like. Among these, a liquid including liquefied gas is preferable in that the decellularized tissue is substantially not damaged and residual liquefied gas is reduced.

Here, a liquid including liquefied gas dissolves the cell membrane components, and can therefore destroy cells of the biological tissue.

The liquefied gas is not limited as long as the cells in the decellularized tissue can be destroyed, and the liquefied gas may be dimethyl ether, ethylmethyl ether, formaldehyde, ketene, acetaldehyde, propane, butane, liquefied petroleum gas, and the like, and two or more of these types may be used in combination. Among these, ethylmethyl ether and dimethyl ether are preferable in being liquefied at relatively low temperature and low pressure, and dimethyl ether is particularly preferable.

Dimethyl ether is liquefied at approximately 1 °C to 40 °C and 0.2 MPa to 5 MPa, and, therefore, the cost of the device will be low. Liquefied dimethyl ether vaporizes readily in the standard state, and is therefore unlikely to remain in the decellularized tissue.

When bringing a liquid including liquefied gas into contact with the biological tissue, this is performed in an environment in which the pressure exceeds the saturated vapor pressure, such as in a gas-tight extraction tank, in order to maintain the liquid state of the liquefied gas.

The method of bringing a liquid capable of destroying cells into contact with the biological tissue may be, but is not limited to, a method of immersing the biological tissue in a liquid capable of destroying cells, etc.

The liquid including liquefied gas may further include a liquid different from the liquefied gas.

The liquid different from liquefied gas may be, but is not limited to, ethanol, water, physiological saline, phosphate buffered saline (PBS), and the like, and two or more kinds of liquids may be used in combination.

The additive amount of the liquid different from liquefied gas is preferably less than or equal to the solubility in the liquefied gas. Accordingly, the liquid including liquefied gas can be made uniform.

The temperature of the liquefied gas is preferably 1 °C to 40 °C, and more preferably 10 °C to 30 °C.

The pressure of the liquefied gas is preferably 0.2 MPa to 5 MPa, and more preferably in the range of 0.3 MPa to 0.7 MPa.

After bringing a liquid including liquefied gas into contact with the biological tissue, the temperature and pressure are returned to the standard state, and then the liquefied gas evaporates, so the liquefied gas can be easily removed.

The step of bringing a liquid including liquefied gas into contact with the biological tissue, may be repeated a plurality of times.

### (Biological tissue-second embodiment)

A biological tissue is defined as a tissue obtained from any one of a plant, fungi, archaea, and eubacteria having cell walls, or an animal without cell walls. Examples of the biological tissue are, but are not limited to, leaves, branches, trees, petals, stems, roots, pulp, pericarp, and seeds; soft tissue including skin, blood vessels, heart valves, cornea, amnion, dura, and the like or portions thereof, organs including heart, kidney, liver, pancreas, brain, and the like or portions thereof, and bones, cartilage, tendon, and the like or portions thereof, derived from humans or heterogeneous mammals.

### [[Practical examples-second embodiment]]

While practical examples of the present invention are described below, the present invention is not limited to the practical examples.

### [Practical example 1-second embodiment]

### (DNA degradation, degreasing, and washing of rat-derived brain tissue in which cells have been destroyed)

Brain tissue derived from a rat in which cells have been destroyed was placed in physiological saline including DNaseI (manufactured by Roche Diagnostics K.K.) and MgCl₂ (manufactured by Wako Pure Chemical Industries, Ltd.) and was shaken, and the DNA, included in the brain tissue derived from a rat in which cells have been destroyed, was degraded. Next, the brain tissue derived from a rat in which the DNA has been degraded, was brought into contact with liquefied dimethyl ether and was degreased. Next, the degreased brain tissue derived from a rat was placed in physiological saline, shaken, and washed. As a result, a decellularized tissue of the brain tissue derived from a rat was obtained.

### [Comparative example 1-second embodiment]

Except that ethanol was used instead of liquefied dimethyl ether when degreasing brain tissue derived from a rat in which the DNA has been degraded, a decellularized tissue of the brain tissue derived from a rat was obtained in the same manner as practical example 1.

Next, the adhesion of cells to the decellularized tissue was evaluated.

### [Cell adhesion of decellularized tissue]

The decellularized tissue from the brain tissue derived from a rat of practical example 1 and comparative example 1, and a brain tissue derived from a rat, were seeded with nerve cells, and after seven days had elapsed, each of these tissues were dyed with fluorescent dye that fluoresces at positions where nerve cells are present, and a cross-section of each sample of these tissues was observed by using a light microscope.

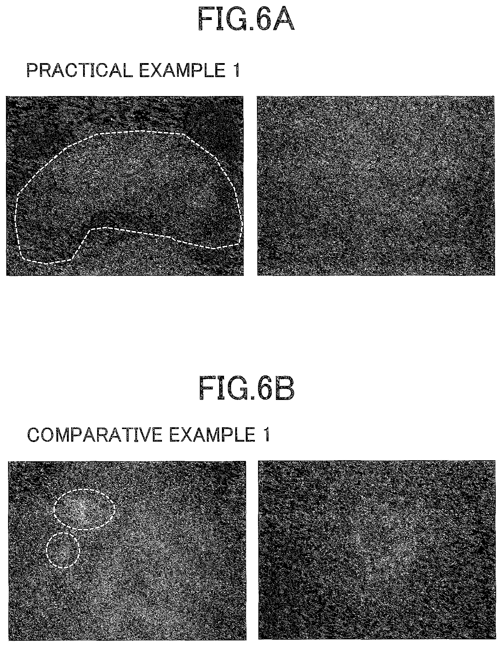

FIGS. 6A and 6B indicate the results of the evaluation of the adhesion of cells to the decellularized tissue. In each of FIGS. 6A and 6B, the left side indicates the decellularized tissue of the brain tissue derived from a rat, and the right side is the brain tissue derived from a rat.

FIGS. 6A and 6B indicate that the decellularized tissue of the brain tissue derived from a rat of practical example 1 has a larger area of fluorescence (dotted line in FIG. 6A) than the decellularized tissue of the brain tissue derived from the rat of comparative example 1. Therefore, it can be seen that by degreasing the brain tissue derived from a rat in which the DNA has been degraded, by using liquefied dimethyl ether, the adhesion of cells to the decellularized tissue can be improved.

### [Practical example 2-second embodiment]

### (Cell destruction of swine-derived aortic tissue)

Cells of aortic tissue 55 derived from a swine were destroyed using the cell destruction apparatus illustrated in FIG. 3.

Specifically, the aortic tissue 55 derived from a swine was charged into an extraction tank 54. Next, the contents in a separation tank 60 were substituted in advance with dimethyl ether, and valves 52, 53, 56, 58, and 59 were closed. Next, the set pressure of a backpressure valve 57 was set to 0.7 MPa, the valves 52, 53, 56, and 58 were opened, the liquefied dimethyl ether was circulated by using a pump 51, and when the extraction tank 54 was filled with the liquefied dimethyl ether, the valves 53 and 56 were closed, the aortic tissue 55 derived from a swine was immersed in the liquefied dimethyl ether, and cell membrane components such as phospholipids were extracted. Next, the valves 53 and 56 were opened, and the flow rate of the liquefied dimethyl ether was adjusted to 1 mL/min, and the extraction liquid was collected in the separation tank 60. Next, the valve 58 was closed, the separation tank 60 was removed from the apparatus, and the liquefied dimethyl ether was volatilized in the draft chamber at atmospheric pressure.

By the above procedure, 60 mL of liquefied dimethyl ether and the aortic tissue 55 derived from a swine were brought into contact with each other, and then the valve 53 was closed, the valves 56, 58, and 59 were opened, the pressure in the extraction tank 54 was set to atmospheric pressure, and the liquefied dimethyl ether in the extraction tank 54 was volatilized and exhausted. As a result, the aortic tissue derived from a swine in which cells have been destroyed, was obtained.

### (DNA degradation, degreasing, and washing of swine-derived aortic tissue in which cells have been destroyed)

The aortic tissue derived from a swine in which cells have been destroyed, was placed in physiological saline containing 0.2 mg/mL of DNaseI (manufactured by Roche Diagnostics K.K.) and 0.05 M of MgCl₂ (manufactured by Wako Pure Chemical Industries, Ltd.), and was shaken to degrade the DNA included in the aortic tissue derived from a swine in which cells have been destroyed. The aortic tissue derived from a swine in which the DNA has been degraded was then brought into contact with liquefied dimethyl ether to be degreased. The degreased aortic tissue derived from a swine was then placed in physiological saline, shaken, and washed. As a result, decellularized tissue of the aortic tissue derived from a swine was obtained.

### [Comparative example 2-second embodiment]

Except that ethanol was used instead of the liquefied dimethyl ether when degreasing the brain tissue derived from a swine in which the DNA has been degraded, a decellularized tissue of the aortic tissue derived from a swine was obtained in the same manner as in practical example 2.

### [Practical examples 3 to 6-second embodiment]

Except that swine-derived cavernous bone tissue, swine-derived skin tissue, swine-derived bone tissue, and swine-derived cartilage tissue were used as the biological tissue, instead of using an aortic tissue derived from a swine, a decellularized tissue was obtained in the same manner as practical example 2 .

### [Comparative examples 3 to 6-second embodiment]

Except that swine-derived cavernous bone tissue, swine-derived skin tissue, swine-derived bone tissue, and swine-derived cartilage tissue were used as the biological tissue, instead of using an aortic tissue derived from a swine, a decellularized tissue was obtained in the same manner as comparative example 2.

The adhesion of cells to the decellularized tissue of practical examples 2 to 6 and comparative examples 2 to 6 was evaluated. In all tissues, it was found that by degreasing the biological tissue derived from a swine in which the DNA has been degraded, by using liquefied dimethyl ether, the adhesion of cells to the decellularized tissue was improved.

The decellularized tissue producing method and the decellularized tissue producing apparatus are not limited to the specific embodiments described in the detailed description, and variations and modifications may be made without departing from the spirit and scope of the present invention.

## Claims

1. A decellularized tissue producing method comprising:
degrading DNA included in a biological tissue in which cells are destroyed, by using a first treatment liquid;
degreasing the biological tissue in which the DNA is degraded, by using a second treatment liquid; and
washing the degreased biological tissue, by using a third treatment liquid, wherein
at least one of the degrading, the degreasing, and the washing is performed by a circulation method.

2. The decellularized tissue producing method according to claim 1, wherein the first treatment liquid is a liquid including an enzyme or a surfactant.

3. The decellularized tissue producing method according to claim 1 or 2, wherein the second treatment liquid is a liquid including an organic solvent.

4. The decellularized tissue producing method according to claim 3, wherein the organic solvent is ethanol.

5. The decellularized tissue producing method according to any one of claims 1 to 4, wherein the third treatment liquid includes water as a main solvent.

6. The decellularized tissue producing method according to any one of claims 1 to 5, further comprising:
destroying the cells in the biological tissue by using a liquid including liquefied gas, wherein
the destroying is performed under a predetermined condition in which the liquefied gas is maintained in a liquid state.

7. The decellularized tissue producing method according to claim 6, wherein the liquefied gas is liquefied dimethyl ether.

8. The decellularized tissue producing method according to claim 7, wherein the predetermined condition is a condition of a temperature of 1 °C to 40 °C and a pressure of 0.2 MPa to 5 MPa.

9. The decellularized tissue producing method according to any one of claims 6 to 8, wherein
at least one of the degrading, the degreasing, the washing, and the destroying is performed in a contact tank, and
the contact tank includes a porous member on an end surface on an upstream side of the contact tank.

10. A decellularized tissue producing apparatus comprising:
a degrader configured to degrade DNA included in a biological tissue in which cells are destroyed, by using a first treatment liquid;
a degreaser configured to degrease the biological tissue in which the DNA is degraded, by using a second treatment liquid; and
a washer configured to wash the degreased biological tissue, by using a third treatment liquid, wherein
at least one of the degrader, the degreaser, and the washer performs a circulation method.

11. A decellularized tissue producing method comprising:
degreasing a biological tissue in which DNA is degraded, by using a liquid including liquefied gas.

12. The decellularized tissue producing method according to claim 11, wherein the degreasing is performed under a predetermined condition in which the liquefied gas is maintained in a liquid state.

13. The decellularized tissue producing method according to claim 12, wherein the predetermined condition is a condition of a temperature of 1 °C to 40 °C and a pressure of 0.2 MPa to 5 MPa.

14. The decellularized tissue producing method according to any one of claims 11 to 13, wherein the liquefied gas is ether.

15. The decellularized tissue producing method according to claim 14, wherein the ether is dimethyl ether.
